# FASCICULE DE BREVET EUROPEEN

(11) **EP 0 839 472 B1**
(45) Date de publication et mention de la délivrance du brevet: **29.05.2002**
(21) Numéro de dépôt: 97402377.2
(22) Date de dépôt: 09.10.1997
(51) Int. Cl.: A45D 40/26

(54) **Applicateur de produit delitable et ensemble d'application comportant un tel applicateur**
Applikator für ein streichfähiges Produkt sowie eine Applikationseinheit mit solch einem Applikator
Applicator for a spreadable product and an application unit comprising such an applicator

(30) Priorité: 31.10.1996 FR 9613360
(43) Date de publication de la demande: 06.05.1998
(73) Titulaire: L'OREAL, 75008 Paris (FR)
(72) Inventeur: Gueret, Jean-Louis H., 75018 Paris (FR)
(74) Mandataire: Boulard, Denis

(56) Documents cités:
- EP-A- 0 526 300
- FR-A- 1 272 557
- FR-E- 53 605
- GB-A- 2 086 849
- US-A- 1 524 008
- US-A- 1 899 386
- US-A- 2 234 558
- US-A- 2 450 919
- US-A- 3 332 429

## Description

La présente invention concerne un applicateur, de type applicateur pour produit de maquillage. Un tel applicateur peut être utilisé notamment pour l'application d'un blush, d'un fard à paupières, d'un fard à joues, d'un rouge à lèvres, d'un fond de teint, d'une crème de soin, d'un produit solaire ou capillaire. D'une manière plus générale, il peut être utilisé pour l'application de tout produit délitable. L'invention concerne également un ensemble d'application, de type boîtier, comportant au moins un applicateur selon l'invention.

On connaît à ce jour différents types d'applicateurs, utilisés notamment pour les produits de maquillage. On connaît entre autres, les applicateurs de type "allumettes" ou "Qtip" se présentant sous forme d'une tige dont une extrémité est chargée par trempage, ou par pistolétage, de produits cosmétiques. Cette première catégorie présente l'inconvénient de déposer trop de produit lors de l'application. Par ailleurs, ce type d'applicateur ne permet pas d'estomper le produit appliqué. Enfin, de par leur configuration, ces applicateurs sont généralement fragiles.

On connaît également des applicateurs, sur la surface desquels un produit de type produit cosmétique est pressé ou compacté. Un des inconvénients majeurs de ces applicateurs tient à leur faible autonomie. Généralement, ces applicateurs sont utilisés comme échantillons.

Enfin, on connaît par ailleurs, des applicateurs de type "houppette" utilisés en combinaison avec un boîtier comportant au moins une coupelle, dans laquelle sont coulés ou compactés des produits cosmétiques. Ces poudriers sont relativement chers.

Le brevet FR 2 642 675 décrit un distributeur de produit solide ou pâteux, contenu dans un réservoir comportant un orifice de distribution, le produit étant poussé vers l'orifice de distribution au moyen d'un mécanisme moteur. Selon ce document, les bords de l'orifice de distribution sont recouverts d'un revêtement tel qu'un flocage ou une mousse de faible épaisseur de manière à permettre l'application du produit. Le dispositif décrit dans ce brevet est d'un fonctionnement complexe, et est relativement coûteux à réaliser.

Il existe également d'autres types d'applicateurs, de type applicateurs à cirage, dans lesquels, le produit à appliquer est contenu dans un tube sur lequel est monté un bloc de mousse percé d'un trou délimitant un canal en communication avec le tube. Dans ces applicateurs, le produit est amené au niveau de la surface d'application, en exerçant une pression sur les parois du tube de manière à contraindre le produit à passer dans le canal de l'applicateur et à l'amener sur la surface d'application. Hormis l'encombrement, ces dispositifs ne sont pas appropriés pour des produits de type produits de maquillage, en raison principalement de la difficulté à doser correctement le produit appliqué.

Les brevets FR 1 272 557, US 1 899 386, et US 2 450 919 décrivent des applicateurs de produit, généralement sous forme de poudre, dans lesquels la poudre est contenue dans un logement ménagé dans un bloc de mousse et dont l'extrémité libre est recouverte d'un tamis, sous forme d'une feuille perforée, d'un tissu, ou autre matériau poreux. Dans ces dispositifs, le produit est libre dans le logement ainsi formé, ce qui rend difficile l'application de façon dosée du produit. Par ailleurs, les orifices, pores, ou autres trous ménagés dans la feuille obturant le logement creux, sont sujets à encrassement rapide, ce qui rend le dispositif inutilisable. Enfin de tels dispositifs ne sont pas adaptés pour des produits de consistance plus élevée, tels que des rouges à lèvres.

Le dispositif décrit dans le brevet US 1 524 008, tout comme ceux décrits dans les documents US 1 899 386 et US 2 450 919, évoqués précédemment, comporte un fond formant partie intégrante du bloc de mousse dans lequel est ménagé le logement. Le savon contenu dans ce logement n'est pas solidaire du logement, ce qui, au fil des utilisations, lui confère une mobilité axiale de plus en plus grande.

Cette mobilité, en particulier axiale, rend l'application du produit très difficile à doser de manière précise, la difficulté à l'application étant encore accentuée par le fait que le fond n'est pas rigide ou semi-rigide. Ces problèmes de précision et dosage de l'application ne sont toutefois pas critiques, lorsque le produit est un savon, comme dans le brevet US 1 524 008.

Aussi, est-ce un des objets de la présente invention que de réaliser un applicateur bon marché, ne présentant pas les inconvénients mentionnés ci-avant, en référence aux applicateurs connus jusqu'alors.

C'est en particulier un objet de l'invention que de fournir un applicateur, d'autonomie suffisante, qui permette de doser de façon satisfaisante le produit appliqué.

C'est un autre objet de l'invention que de réaliser un applicateur, qui, outre la fonction d'application, permette d'estomper le produit appliqué.

C'est encore un autre objet de la présente invention que de fournir un applicateur contenant de manière intégrée, une réserve du produit à appliquer, et permettant une autonomie, une précision, une qualité, et une douceur d'application aussi satisfaisantes que celles de dispositifs plus onéreux dans lesquels le produit est appliqué au moyen d'un dispositif d'application utilisé en combinaison avec une réserve de produit séparée.

D'autres objets de l'invention apparaîtront de manière détaillée dans la description qui suit.

Selon l'invention, ces objets sont atteints en réalisant un applicateur de produit délitable comportant au moins un élément déformable élastiquement dont au moins une face présente une surface d'application du produit, l'un au moins des éléments élastiquement déformables présentant au moins un trou d'axe A, débouchant sur la surface d'application, et délimitant au moins un logement dans lequel est disposé fixement le produit, ledit produit présentant au moins une surface libre, ledit élément déformable élastiquement pouvant passer sélectivement d'une position de repos dans laquelle ladite surface libre se situe au niveau, ou en dessous, de la surface d'application, à une position d'application dans laquelle l'élément élastiquement déformable est au moins partiellement comprimé selon l'axe A, et dans laquelle ladite surface libre est sensiblement au niveau de la surface d'application.

Au sens de la présente demande, l'expression "un trou d'axe A, débouchant sur la surface d'application" traduit le fait que le trou débouche sur la surface d'application, et ce sur la totalité de sa section. En d'autres termes, en position d'application, il n'est pas recouvert par un tamis ou autre élément perforé ou poreux, apte à l'isoler de la surface sur lequel est appliqué le produit.

De même, l'expression "disposé fixement" traduit le fait que le produit est sensiblement immobile axialement dans l'applicateur, et que l'application se fait par déplacement (compression/décompression) de la surface de l'élément élastiquement déformable qui entoure le produit, lequel déplacement contribue sélectivement à "découvrir" le produit et à le faire émerger sensiblement au niveau de la surface d'application, en vue de son application directe sur une surface à traiter. Le produit peut être immobilisé axialement, soit grâce à des moyens d'ancrage prévus sur un fond du logement formé par exemple, par une âme rigide ou semi rigide, soit par ancrage latéral du produit dans la structure du matériau (les pores d'une mousse par exemple) constituant le (ou les) élément(s) élastiquement déformables(s), soit les deux.

Ainsi, en vue d'une application de produit, on dispose l'applicateur sur la surface à traiter. En exerçant une pression sur l'applicateur, l'élément élastiquement déformable délimitant le logement, se comprime au moins partiellement, de sorte que le produit vienne au contact de la surface à traiter. A ce moment, par des petits mouvements circulaires par exemple, on dépose du produit sur la surface. En relâchant la pression exercée sur l'applicateur, la surface libre du produit rentre à l'intérieur du logement. Seule la surface d'application de l'élément élastiquement déformable reste en contact avec la surface à traiter. Cette surface libre peut alors être utilisée pour parfaire l'étalement du produit sans rajouter de produit, et/ou estomper le produit.

Le fait que le produit soit maintenu fixement à l'intérieur du logement, avantageusement, en formant le fond du logement contenant le produit, à partir d'une âme rigide ou semi-rigide, contribue à réaliser un dosage encore plus précis du produit appliqué.

Ainsi que mentionné précédemment, l'applicateur comporte des moyens d'ancrage de manière à maintenir, au moins axialement, le produit à l'intérieur du logement. Les moyens d'ancrage peuvent être situés sur les parois du logement et/ou sur le fond du logement. Le produit peut être coulé, ou compacté à l'intérieur du compartiment. Il peut être également coulé ou compacté préalablement dans une cupule, et monté ensuite (avec la cupule) dans le logement.

Selon l'invention, le bloc de mousse peut être comprimé sur une hauteur telle que quel que soit le niveau de produit dans le logement, la surface d'application puisse être abaissé au niveau de la surface libre du produit, de manière à permettre l'application de sensiblement tout le produit, le produit étant disposé fixement dans le logement. En d'autres termes, le produit est amené au niveau de la surface d'application en abaissant la surface d'application, par compression de l'élément élastiquement déformable, au niveau de la surface du produit dans l'évidement. Le produit ne peut pas coulisser de manière sensible à l'intérieur du logement.

Le logement peut déboucher uniquement sur une première surface d'application, ou peut déboucher également sur une seconde surface d'application, opposée à la première surface d'application. Dans cette dernière configuration, le logement peut contenir un premier et un second produits, le premier produit étant dans la partie du logement débouchant sur la première surface d'application, le second produit étant dans la partie du logement débouchant sur la seconde surface d'application. Lesdits premier et second produits peuvent être de même couleur, ou de couleur différente. Dans cette hypothèse, l'ancrage du produit se fait sur les parois latérales du logement, par exemple dans les pores d'une mousse à cellules ouvertes. En effet, lors du coulage, le produit pénètre dans les pores de la mousse, situées au voisinage des bords latéraux délimitant le logement. En durcissant, le produit ainsi retenu dans les cellules ouvertes de la mousse, immobilise le bloc de produit dans son logement.

L'élément élastiquement déformable est monté avantageusement sur un organe rigide ou semi rigide dont une première extrémité définit un fond pour ledit logement, une seconde extrémité définissant un moyen de préhension pour ledit applicateur.

Avantageusement, l'applicateur comporte au moins un bloc de mousse à cellules ouvertes, semi ouvertes, ou fermées. Dans le cas d'une mousse à cellules ouvertes ou semi ouvertes, les cellules constituent les moyens d'ancrage latéraux pour le produit. A titre d'exemple, on utilise une mousse de chlorure de polyvinyle (PVC), de polyuréthanne, de polyéther, de polyester, ou d'un élastomère de type SBR (synthetic butadiène rubber), NBR, silicone, nitrile, etc.. Selon une alternative, les moyens d'accrochage sont constitués d'éléments tels que des ailettes ou des nervures ménagées dans le fond du logement.

Selon un mode de réalisation, l'applicateur comporte deux blocs de mousse à cellules ouvertes ou semi ouvertes, un premier bloc définissant au moins un logement pour le produit, un second bloc disposé sur la surface du premier bloc opposée à la surface d'application. Les deux blocs de mousse sont séparés par une âme rigide ou semi rigide, l'un des blocs au moins définissant au moins un logement pour ledit produit, ladite âme formant un fond pour le (ou les) logement(s).

Selon une alternative, l'applicateur comporte un bloc de mousse à cellules ouvertes ou semi ouvertes, dont une face, opposée à la surface d'application est montée sur une âme rigide ou semi rigide, ladite âme formant un fond pour ledit logement.

La surface d'application peut être recouverte d'un flocage ou d'un matériau textile, ou d'une trame de manière à favoriser l'application du produit, et d'en augmenter la douceur.

Le produit peut être un blush, un fard à joues, un fard à paupières, un rouge à lèvres, un fond de teint, un produit de soin, un produit solaire ou capillaire, etc..

Selon un autre aspect de l'invention, on réalise un ensemble d'application comportant :
a) un boîtier présentant un fond et un couvercle apte à recouvrir ledit fond; et
b) au moins un applicateur monté dans ledit boîtier, le (ou les) applicateur(s) étant conforme(s) à l'invention.

Le fond du boîtier peut être agencé de manière à définir un fond pour le (ou les) trou(s) dudit applicateur. De la même manière, le couvercle peut présenter un orifice qui, en position fermée du couvercle sur le fond, est situé sensiblement en regard du trou de l'applicateur, de manière à permettre de couler le produit dans l'applicateur au travers dudit orifice. Cet orifice peut, après coulage du produit dans l'applicateur, être recouvert d'une étiquette transparente de manière à permettre de visualiser la couleur du produit dans le boîtier.

L'invention consiste, mise à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- les figures 1A et 1B illustrent un premier mode de réalisation d'un applicateur selon l'invention et dans lequel, le produit est disposé dans l'applicateur par coulage;
- les figures 2 et 3 représentent un second mode de réalisation d'un applicateur selon l'invention;
- les figures 4 et 5 illustrent un troisième mode de réalisation de l'applicateur selon l'invention;
- la figure 6 représente un quatrième mode de réalisation d'un applicateur selon l'invention;
- les figures 7A-7D illustrent un premier mode de réalisation d'un ensemble d'application comportant un applicateur selon l'invention;
- les figures 8A-8E illustrent des variantes du mode de réalisation des figures 7A-7D;
- les figures 9 à 11 illustrent de façon schématique d'autres modes de réalisation d'un ensemble d'application selon l'invention; et
- la figure 12 représente de façon partielle un boîtier comportant une pluralité d'applicateurs selon l'invention.

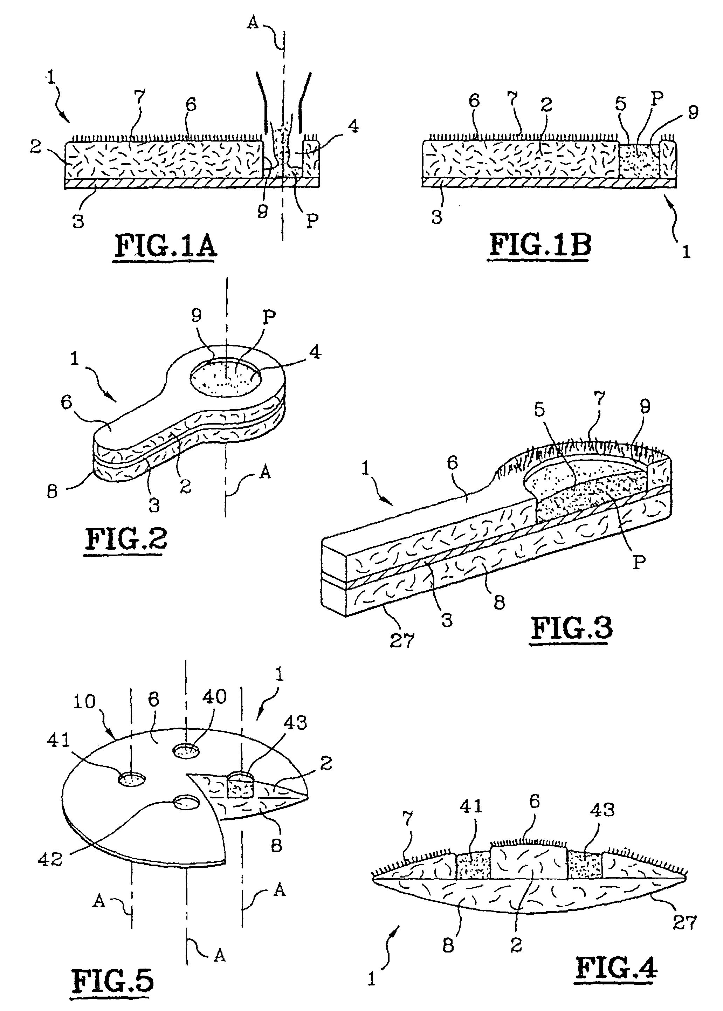

L'applicateur 1 représenté aux figures 1A et 1B comporte un bloc de mousse 2, monté par exemple sur un support rigide ou semi rigide 3. Le bloc de mousse comporte un trou 9 d'axe A, définissant un logement 4 pour le produit P, le support rigide ou semi rigide 3 formant un fond pour ledit logement. La hauteur, la densité, et l'élasticité du ou des bloc(s) de mousse sont choisies de manière appropriée, de sorte que, en position de repos, le produit soit sensiblement contenu à l'intérieur du logement 4 (au maximum, au niveau de la surface d'application avant la première utilisation du produit) et de sorte que sensiblement tout le produit contenu dans l'applicateur puisse être utilisé. Typiquement, pour un rouge à lèvres, on coule le produit sur une hauteur comprise entre 2 mm et 14 mm, pour un logement dont la profondeur au repos est comprise entre 2 mm et 15 mm.

Dans le mode de réalisation représenté, le produit est introduit et ancré dans le logement par coulage du produit sous forme liquéfié qui se transforme, après solidification, en un produit solide et délitable. Le produit liquéfié peut être présenté sous forme de pâte, obtenue en mélangeant une phase particulaire solide, soit avec une phase aqueuse, soit avec un liant, notamment une phase grasse dans un solvant. Elle peut également être sous forme d'un produit à base de cires thermofusibles ou d'un gel qui est coulé à l'état chaud. Selon le type de composition choisi, la solidification se fait donc, soit par évaporation d'eau ou de solvant, soit par refroidissement, soit par réaction chimique. Le produit peut être une pâte anhydre, coulée à chaud. Alternativement, le produit à couler contient, outre des pigments colorés, de charges et de liants, du sulfate de calcium hemihydrate (CaSO₄, ½ H₂O) et une quantité suffisante d'eau pour obtenir un mélange coulable, la solidification se produisant, après coulage dans le trou, par formation de sulfate de calcium dihydrate (CaSO₄, 2H₂O). Selon une alternative, le produit P peut être contenu dans le logement sous forme compactée.

Le bloc de mousse peut être constitué de mousse à cellules ouvertes, semi ouvertes, ou fermées. A titre d'exemple, on utilise une mousse de chlorure de polyvinyle (PVC), de polyuréthanne, de polyéther, de polyester, ou d'un élastomère de type SBR (synthetic butadiène rubber), NBR, silicone, nitrile, etc.. La mousse peut être montée au dessus d'une paroi souple déformable moulée en thermoplastique ou en élastomère.

Le fond et/ou les parois latérales du logement peuvent être étanches au produit P. L'étanchéité peut être obtenue par tout moyen connu approprié, en utilisant, soit par exemple, une mousse à cellules fermées, soit en déposant sur les parois du trou un revêtement étanche au produit P. Alternativement, le bloc de mousse est obtenu par moulage d'un matériau tel que du latex, le produit résultant du moulage présentant des parois parfaitement étanches, y compris les parois du logement 4.

Le fond et/ou les parois latérales du logement 4 comportent des moyens destinés à permettre l'accrochage du produit dans le logement, et à l'immobiliser axialement. Selon un mode de réalisation préféré, l'ancrage du produit dans le logement est réalisé en utilisant une mousse à cellules ouvertes ou semi ouvertes. Avec une telle configuration, lorsque le produit est coulé dans le logement, il s'introduit dans les cellules situées principalement au voisinage des parois du logement. Après solidification, le produit P est immobilisé dans le logement 4. Comme on le verra plus en détail par la suite, l'ancrage du produit P dans le logement se fait de préférence par le fond de ce dernier, ou en tout cas, par la partie inférieure du logement, afin de ne pas gêner sensiblement la compression de l'élément élastiquement déformable.

Le bloc de mousse 2 présente une surface d'application 6 du produit, qui, selon le mode de réalisation représenté, est recouverte d'un flocage 7. Alternativement, la surface d'application peut être recouverte d'un autre matériau, tel qu'un matériau textile, ou une trame, le choix de la nature de la surface d'application dépendant du type d'application du produit.

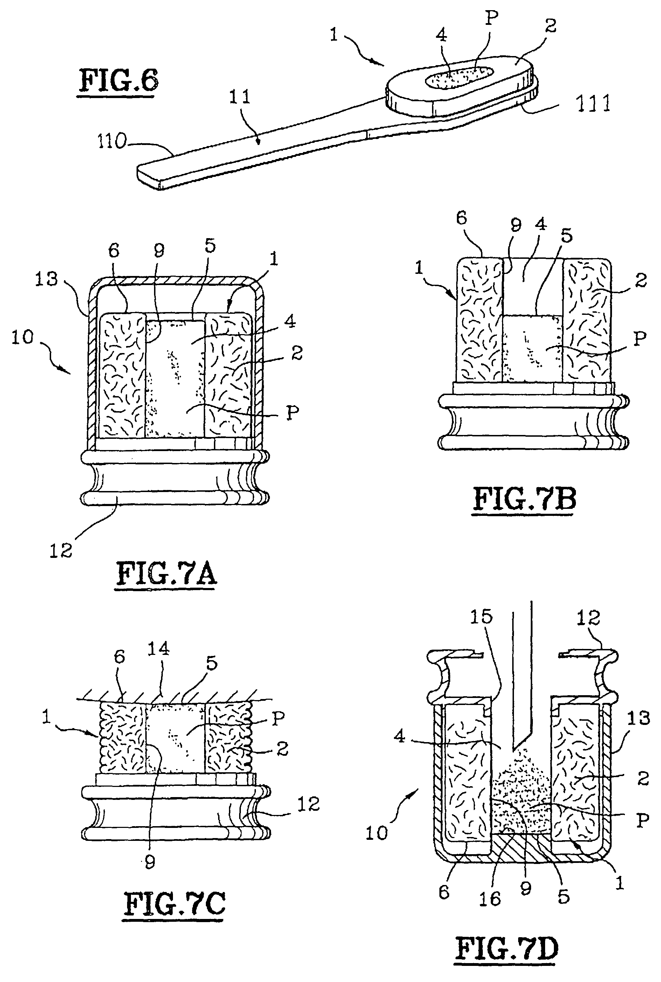

Ainsi que représenté à la figure 1B, en position de repos (non comprimée) de l'élément élastiquement déformable 2, la surface libre 5 du produit P se trouve au niveau (lorsque l'applicateur n'a pas encore été utilisé) ou de préférence en dessous de la surface d'application, de sorte que, en position de repos, le produit soit sensiblement protégé par la mousse qui l'entoure. Comme on le verra plus en détail par la suite en référence à la figure 6C, lors de l'utilisation du produit, l'applicateur est appliqué sur la peau, ce qui provoque une compression (au moins partielle) de la mousse jusqu'à ce que la surface libre du produit soit sensiblement au niveau de la surface d'application. Le produit est alors en contact de la peau, la surface d'application permettant une bonne répartition du produit sur la peau. Après avoir relâché la pression exercée sur l'applicateur, l'utilisatrice peut ensuite estomper le produit, soit avec la surface d'application, soit avec une autre surface de l'applicateur. Le produit peut ainsi être dosé et appliqué parfaitement, en fonction de la pression exercée sur l'applicateur, et en fonction de la durée pendant laquelle la pression est exercée. Au fil des utilisations, le niveau de produit dans le logement diminue, jusqu'à utilisation sensiblement complète du produit.

Selon une autre alternative, le moyen élastiquement déformable est constitué d'un système à soufflet entourant de manière étanche, le logement 4, et dont une surface, est recouverte d'un revêtement approprié de manière à conférer à l'application du produit, la qualité et la douceur souhaitées.

De même, selon une autre alternative encore, les orifices ménagés dans le bloc de mousse élastique peuvent être de section autre que circulaire. A titre d'exemple, le (ou les) orifices définissent un ou plusieurs logements concentriques dans le bloc de mousse.

Les figures 2 et 3 auxquelles il est maintenant fait référence illustrent un second mode de réalisation de l'applicateur selon l'invention, pouvant être utilisé comme un applicateur de type ombre à paupières. Il présente une partie sensiblement circulaire à l'intérieur de laquelle est disposé le logement 4 pour le produit P, et une partie de forme allongée destinée à la préhension de l'applicateur, soit à la main, soit au moyen d'un dispositif tel qu'une pince (non représentée). Selon ce mode de réalisation, un second bloc de mousse 8 est disposé de l'autre coté du support rigide ou semi-rigide 3, et comporte une surface 27, opposée à la surface d'application 6 qui permet par exemple d'estomper le produit appliqué par l'intermédiaire de la surface 6.

Ainsi qu'illustré en coupe à la figure 3, la surface d'application 6 du produit comporte un flocage 7, ou tout autre revêtement (textile, élastomère, etc.) permettant d'améliorer la douceur et la qualité de l'application.

Dans le mode de réalisation illustré aux figures 4 et 5, l'applicateur se présente sous forme d'une houppette 1 de forme bombée sur ses deux faces principales, et comportant deux parties en mousse. La première partie 2 est percée de plusieurs orifices (en l'occurrence 4) définissant une pluralité de logements 40, 41, 42, 43 pouvant contenir des produits identiques ou différents. Tous les orifices débouchent sur la surface d'application 6 de l'applicateur. La partie inférieure 8 de l'applicateur est constituée de mousse, laquelle, avantageusement, est séparée de la première 2 par une plaque rigide ou semi-rigide (non représentée) et définissant un fond pour les logements 40, 41, 42, 43. Dans cette configuration, l'accrochage du produit dans les logements 40, 41, 42, 43 peut se faire par le fond. La face 27 opposée à la surface d'application peut être utilisée pour estomper le produit appliqué par la surface d'application 6. Ainsi qu'il apparaît à la figure 4, la surface d'application peut également être recouverte d'un revêtement de flocage 7.

Dans le mode de réalisation de la figure 6, l'applicateur est monté sur une extrémité d'un élément de forme allongée 11, sensiblement plan. L'élément 11 constitue en l'une de ses extrémités 111, un fond pour le logement 4 ménagé dans un bloc de mousse 2. L'autre extrémité 110 de l'organe forme un élément de préhension de manière à faciliter la manipulation de l'applicateur par l'utilisatrice. De préférence, l'élément 11 est constitué d'un matériau rigide ou semi rigide.

Les figures 7A-7D auxquelles il est maintenant fait référence représentent un applicateur monté dans un boîtier 10 selon un mode de réalisation de l'invention. Celui-ci comprend principalement un fond 12, sur lequel est monté, par exemple par collage un bloc de mousse 2 dans lequel est ménagé un orifice définissant un logement 4 pour le produit P. Le fond du boîtier forme également le fond du logement 4. Un couvercle 13 est monté de manière amovible sur le fond. Dans la configuration représentée à la figure 7A, avant le première utilisation, la surface libre 5 du produit se trouve au niveau, ou légèrement en dessous de la surface d'application 6. Ainsi que mentionné précédemment, l'accrochage du produit peut se faire par l'intermédiaire des cellules ouvertes ou semi ouvertes du bloc de mousse 2. Alternativement, l'accrochage du produit, en particulier au fond du logement, peut être réalisé par d'autres moyens, tels que des ailettes, des nervures, ou d'autres reliefs appropriés, ménagés sur la surface du fond du boîtier sur laquelle est monté le bloc de mousse.

A la figure 7B, après plusieurs utilisations, la surface libre 5 du produit P se trouve sensiblement en dessous de la surface d'application. Le produit est ainsi protégé à l'intérieur du logement 4.

Dans la position de la figure 7C, l'applicateur est appliqué sur la peau 14. Le bloc de mousse est partiellement comprimé de sorte que la surface libre 5 du produit P soit sensiblement au niveau de la surface d'application 6. Le produit peut alors être appliqué, de manière dosée sur la surface de la peau.

La figure 7D illustre un mode de remplissage du boîtier selon l'invention. Dans ce mode de réalisation, le remplissage s'effectue par le fond du boîtier au travers d'un orifice 15, après avoir retourné le boîtier tête en bas. L'orifice 15 est, après remplissage, obturé au moyen d'une pastille, qui peut être soudée, claquée, ou collée sur le fond. Le couvercle est utilisé comme fond lors du coulage du produit. Il comporte une partie centrale 16 de diamètre sensiblement égal au diamètre du logement 4, pour former un fond lors du coulage du produit P, et limiter le remplissage du logement 4, à un niveau de préférence légèrement en dessous de la surface d'application, lorsque celle ci est au repos. Avantageusement, la surface libre 5 du produit est, avant la première utilisation, de 1 à 5 mm en dessous du niveau de la surface d'application 6.

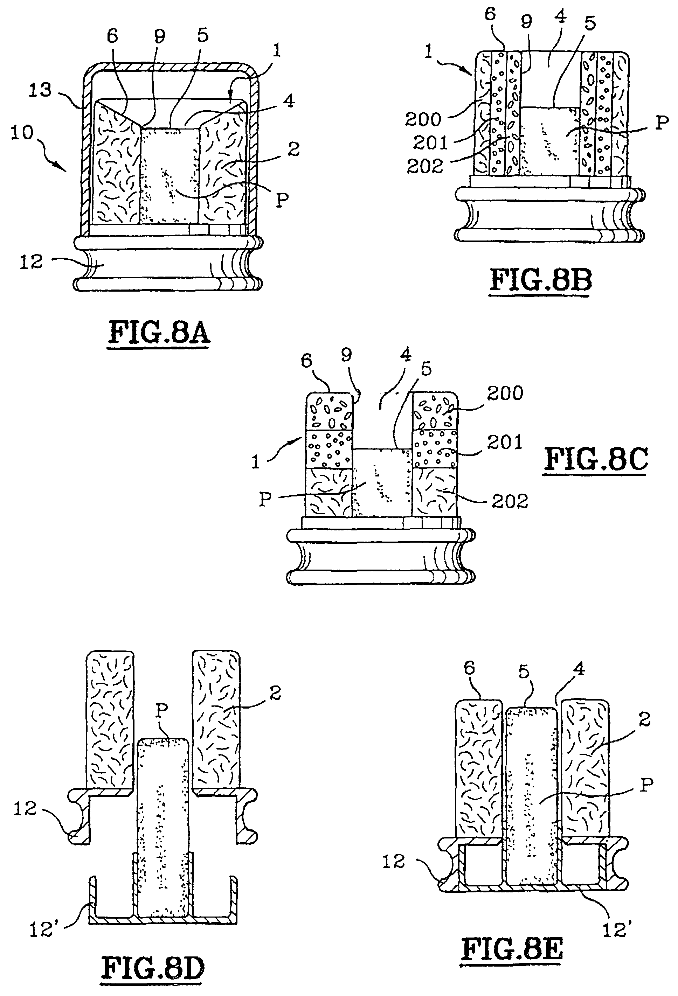

Dans le mode de réalisation de la figure 8A, la surface d'application 6 présente au repos, un profil tronconique, dont la partie de plus faible diamètre est adjacente au logement 4. Une telle configuration permet d'obtenir des variantes et des nuances dans l'application du produit. Lors de l'application du produit, la surface d'application s'écrase et devient sensiblement plane de manière à épouser la surface de la peau.

Ainsi qu'illustré à la figure 8B, le bloc de mousse peut se présenter sous forme d'une pluralité de portions concentriques 200, 201, 202 autour du logement 4, chacun des blocs pouvant présenter des duretés ou des densités différentes, de manière à permettre des nuances dans l'application du produit.

Dans le mode de réalisation de la figure 8C, l'élément élastiquement déformable est réalisé sous forme d'un tube formé d'un empilement de plusieurs blocs de mousse superposés, chacun des blocs étant traversé par un trou central. Les blocs 200, 201, 202, sont disposés de sorte que leurs trous centraux respectifs soient sensiblement alignés. De la même manière que pour le mode de réalisation de la figure 8B, chacun des blocs peut présenter des duretés ou des densités différentes. L'empilement peut être obtenu par moulage ou par collage des différents blocs empilés.

Dans le mode de réalisation de la figure 8D, le produit est par exemple un rouge à lèvres, monté (coulé par exemple) préalablement dans une cupule 12'. L'ensemble est monté fixement dans l'applicateur, au travers d'un orifice formé dans son fond 12. L'ensemble monté est représenté à la figure 8E. La cupule 12', peut être soudée, claquée ou collée sur le fond 12. Avantageusement, la cupule 12' présente des moyens, telles que des ailettes pour permettre l'ancrage du produit dans la cupule.

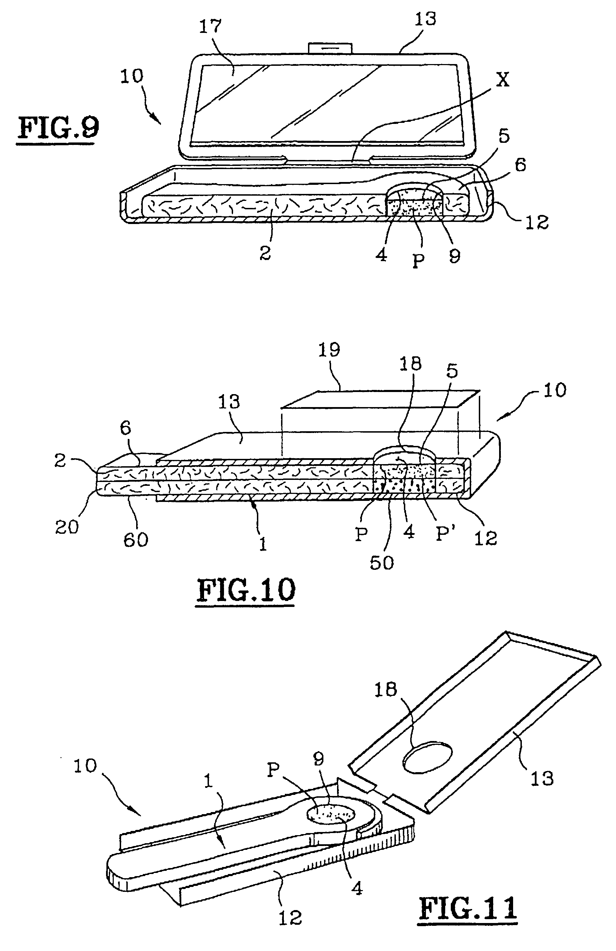

Le boîtier de la figure 9 comporte un couvercle 13 articulé sur le fond, autour d'un axe d'articulation X. La surface interne du couvercle comporte un miroir 17. Selon ce mode de réalisation, le remplissage du logement 4 se fait par le dessus, le fond du boîtier servant de fond au logement 4.

Dans le mode de réalisation de la figure 10, l'applicateur comporte deux faces d'application 6, 60. L'applicateur comporte deux blocs de mousse superposés 2, 20, séparés éventuellement par une âme rigide ou semi rigide. Le logement traverse de part en part les deux blocs 2, 20, de sorte que le logement 4 débouche à la fois sur une première surface d'application 6, et sur une seconde surface d'application 60, opposée à la première. Le boîtier se présente sous forme d'un étui dont une partie forme un fond 12, et une autre partie forme un couvercle. Le couvercle comporte un orifice 18, qui, lorsque l'applicateur est inséré dans l'étui, se situe sensiblement en alignement avec le logement 4, de sorte que le remplissage peut se faire par coulage au travers de l'orifice 18, le fond de l'étui définissant un fond pour le logement, lors du coulage du produit, ou des produits. En effet, selon ce mode de réalisation, dans la partie du logement débouchant sur la surface 60, on coule un premier produit P'. Dans la partie du logement débouchant sur l'autre surface d'application 6, on coule ensuite un second produit P. A titre d'exemple, on utilise des produits de couleurs différentes. Ainsi, on réalise un applicateur bi-faces. L'immobilisation axiale des produits P, P' se fait par ancrage du produit dans les cellules ouvertes du matériau dans lequel est ménagé le logement 4. L'étui peut être réalisé en un matériau translucide de manière à visualiser la couleur des produits contenus dans l'applicateur. Alternativement, on colle sur le couvercle 13, une étiquette translucide 19, de manière à obturer l'orifice 18 après remplissage, et à permettre également la visualisation de la couleur du produit P. Alternativement, l'applicateur selon cette configuration est réalisé au moyen d'un seul bloc de mousse, dont l'épaisseur est choisie de manière appropriée.

Dans le mode de réalisation de la figure 11, le couvercle est articulé sur le fond 12. De la même manière que pour le mode de réalisation la figure 9, le couvercle comporte un orifice qui, en position fermée du couvercle 13 sur le fond 12, est sensiblement en alignement avec le logement 4. Le logement 4 peut ainsi être rempli au travers de l'orifice 18.

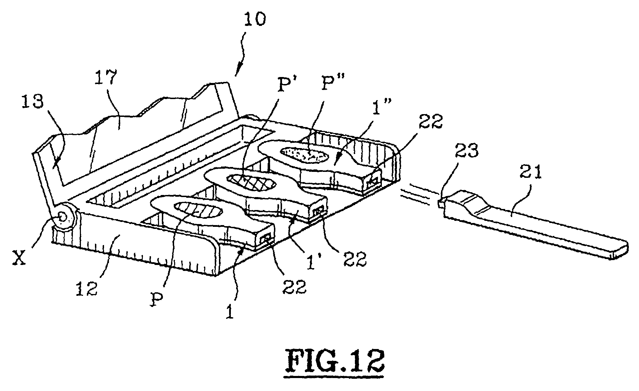

A la figure 12, l'ensemble d'application 10, comporte une pluralité d'applicateurs 1, 1', 1", comportant chacun un produit différent P, P', P". L'ensemble est monté à l'intérieur d'un boîtier comportant un couvercle 13, articulé sur un fond 12 autour d'un axe d'articulation X. La surface interne du couvercle comporte un miroir 17. Chacun des applicateurs comporte une partie femelle 22 apte à coopérer avec une partie mâle 23 d'un manche 21. Un logement 24 est ménagé dans le fond du boîtier 10 de manière à recevoir le manche 21. Ainsi, on réalise un kit, de maquillage par exemple, comportant une pluralité d'applicateurs, pouvant être utilisés à volonté au moyen d'un même manche. L'ensemble est compact, de petite taille et peut être transporté aisément dans un sac à main.

L'invention qui vient d'être décrite est particulièrement avantageuse, en ce qu'elle réalise un applicateur, de type applicateur de maquillage, comportant de manière intégré une réserve du produit à appliquer, et offrant toutes les possibilités et caractéristiques des applicateurs conventionnels, en termes d'autonomie, de qualité, précision, et douceur d'application.

Dans la description détaillée qui précède, il a été fait référence à des modes de réalisation préférés l'invention. Il est évident que des variantes peuvent y être apportées sans s'écarter de l'esprit de l'invention telle que revendiquée ci-après.

## Revendications

1. Applicateur (1) de produit délitable (P) comportant au moins un élément déformable élastiquement (2, 8) dont au moins une face constitue une surface d'application (6, 60) du produit, l'un au moins des éléments élastiquement déformables présentant au moins un trou (9) d'axe A, **caractérisé en ce que** le trou débouche sur la surface d'application (6) sur la totalité de sa section, et délimite au moins un logement ouvert (4, 40, 41, 42, 43) dans lequel est disposé fixement le produit, ledit produit présentant au moins une surface libre (5, 50), ledit élément déformable élastiquement pouvant passer sélectivement d'une position de repos dans laquelle ladite surface libre (5, 50) se situe au niveau, ou en dessous, de la surface d'application (6, 60), à une position d'application dans laquelle l'élément élastiquement déformable (2, 8) est au moins partiellement comprimé selon l'axe A, et dans laquelle ladite surface libre (5, 50) est sensiblement au niveau de la surface d'application (6, 60).

2. Applicateur selon la revendication 1 **caractérisé en ce que** le produit est coulé ou compacté à l'intérieur du logement.

3. Applicateur selon la revendication 1 **caractérisé en ce que** le produit est monté dans le logement par l'intermédiaire d'une cupule (12').

4. Applicateur selon la revendication 1 ou 2 **caractérisé en ce qu'**il comporte une surface (3) opposée à la surface d'application (6), ledit logement (4) débouchant uniquement sur la surface d'application.

5. Applicateur selon la revendication 1 ou 2 caractérisé en qu'il comporte une seconde surface d'application (60) opposée à une première surface d'application (6), ledit logement débouchant à la fois sur la première surface d'application (6) et sur la seconde surface d'application (60).

6. Applicateur selon la revendication 5 **caractérisé en ce que** le logement contient un premier et un second produit (P, P'), le premier produit (P) étant dans la partie du logement débouchant sur la première surface d'application (6), le second produit (P') étant dans la partie du logement (4) débouchant sur la seconde surface d'application (60).

7. Applicateur selon la revendication 6 **caractérisé en ce que** lesdits premier et second produits (P, P') sont de couleur différente.

8. Applicateur selon la revendication 1, **caractérisé en ce qu'**il comporte un élément élastiquement déformable monté sur un organe rigide ou semi rigide (11) dont une première extrémité (111) définit un fond pour ledit logement (4), une seconde extrémité (110) définissant un moyen de préhension pour ledit applicateur (1).

9. Applicateur selon l'une quelconque des revendications précédentes **caractérisé en ce que** le produit (P) est immobilisé dans le (ou les) logement(s) (4, 40, 41, 42, 43), par des moyens d'ancrage situés sur le fond et/ou sur les parois du logement.

10. Applicateur selon l'une quelconque des revendications 1 à 9 **caractérisé en ce qu'**il comporte au moins un bloc de mousse à cellules ouvertes, semi ouvertes, ou fermées (2, 8, 20).

11. Applicateur selon la revendication 10 **caractérisé en ce qu'**il comporte au moins un bloc de mousse à cellules ouvertes ou semi ouvertes, lesdites cellules constituant les moyens d'ancrage pour le produit.

12. Applicateur selon la revendication 10 **caractérisé en ce qu'**il comporte deux blocs (2, 8) de mousse à cellules ouvertes ou semi ouvertes, un premier bloc (2) définissant au moins un logement (4) pour le produit, un second bloc (8) définissant un fond pour ledit logement et une surface (27) pour estomper le produit appliqué au moyen de la surface d'application (6), les deux blocs (2, 8) étant séparés par une âme rigide ou semi rigide (3), ladite âme formant un fond pour ledit logement (4).

13. Applicateur selon la revendication 10 ou 11 **caractérisé en ce qu'**il comprend une pluralité de blocs de mousse concentriques (200, 201, 202) disposés autour du logement (4).

14. Applicateur selon la revendication 10 ou 11 **caractérisé en ce qu'**il comporte un empilement d'une pluralité de blocs de mousse (200, 201, 202), chacun des blocs de mousse présentant un trou le traversant de part en part, les blocs de mousse étant empilés de sorte que les trous de chacun des blocs de mousse (200, 201, 202) soient sensiblement alignés.

15. Applicateur selon l'une quelconque des revendications précédentes **caractérisé en ce que** la surface d'application (6) présente, en position de repos de l'élément élastiquement déformable (2), un profil tronconique, dont le plus petit diamètre est adjacent au logement (4).

16. Applicateur selon l'une quelconque des revendications 1 à 15 **caractérisé en ce que** la (ou les) surface(s) d'application (6, 60) est (sont) recouverte(s) d'un flocage (7), d'un matériau textile, ou d'une trame.

17. Applicateur selon l'une quelconque des revendications précédentes **caractérisé en ce que** le (ou les) élément(s) élastiquement déformable(s) (2, 8) est (sont) constitué(s) d'une mousse de chlorure de polyvinyle (PVC), de polyuréthanne, de polyéther, de polyester, ou d'un élastomère de type SBR (synthetic butadiène rubber), NBR, silicone, nitrile, etc..

18. Applicateur selon l'une quelconque des revendications précédentes **caractérisé en ce que** ledit produit (P, P', P") est un blush, un fard à joues, un fard à paupières, un rouge à lèvres, un fond de teint, un produit de soin, un produit solaire, ou un produit capillaire, etc..

19. Ensemble d'application (10) comportant :
a) un boîtier présentant un fond (12) et un couvercle (13) apte à recouvrir ledit fond; et
b) au moins un applicateur (1, 1', 1") monté dans ledit boîtier,
**caractérisé en ce que** le (ou les) applicateur(s) est(sont) conforme(s) à l'une quelconque des revendications précédentes.

20. Ensemble d'application selon la revendication 19, **caractérisé en ce que** le fond (12) du boîtier définit un fond pour le (ou les) logement(s) (4) dudit applicateur.

21. Ensemble d'application selon la revendication 19, **caractérisé en ce que** le couvercle présente un orifice (18) qui, en position fermée du couvercle (13) sur le fond (12), est situé sensiblement en regard du trou (9) de l'applicateur, de manière à permettre de couler le produit dans l'applicateur au travers dudit orifice (18).

22. Ensemble d'application selon la revendication 21 **caractérisé en ce que** l'orifice (18) du couvercle est, après coulage du produit (P) dans l'applicateur, recouvert d'une étiquette transparente (19) de manière à permettre de visualiser la couleur du produit (P) dans le boîtier.

## Patentansprüche

1. Applikator (1) für ein ablösbares Produkt (P) , der mindestens ein elastisch verformbares Element (2, 8) enthält, von dem mindestens eine Seite eine Auftragfläche (6, 60) für das Produkt aufweist, wobei mindestens eines der elastisch verformbaren Elemente mindestens ein Loch (9) mit einer Achse A aufweist, **dadurch gekennzeichnet, daß** das Loch an der Auftragfläche (6) über seinen ganzen Querschnitt mündet und mindestens einen offenen Raum (4, 40, 41, 42, 43) begrenzt, in dem das Produkt fest angeordnet ist, wobei das Produkt mindestens eine freie Oberfläche (5, 50) aufweist, wobei das elastisch verformbare Element wahlweise von einer Ruhestellung, in der die freie Oberfläche (5, 50) sich in Höhe oder unter der Ruftragfläche (6, 60) befindet, in eine Auftragstellung übergehen kann, in der das elastisch verformbare Element (2, 8) zumindest teilweise entlang der Achse A zusammengedrückt wird, und in der die freie Oberfläche (5, 50) sich im wesentlichen in Höhe der Auftragfläche (6, 60) befindet.

2. Applikator nach Anspruch 1, **dadurch gekennzeichnet, daß** das Produkt in den Raum gegossen oder in ihm verdichtet wird.

3. Applikator nach Anspruch 1, **dadurch gekennzeichnet, daß** das Produkt in den Raum mit Hilfe eines Bechers (12') eingesetzt wird.

4. Applikator nach Anspruch 1 oder 2, **dadurch gekennzeichnet, daß** er eine Fläche (3) entgegengesetzt zur Auflagefläche (6) aufweist, wobei der Raum (4) nur an der Auftragfläche mündet.

5. Applikator nach Anspruch 1 oder 2, **dadurch gekennzeichnet, daß** er eine zweite Auftragfläche (60) entgegengesetzt zu einer ersten Auftragfläche (6) aufweist, wobei der Raum sowohl an der ersten Auftragfläche (6) als auch an der zweiten Auftragfläche (60) mündet.

6. Applikator nach Anspruch 5, **dadurch gekennzeichnet, daß** der Raum ein erstes und ein zweites Produkt (P, P') enthält, wobei das erste Produkt (P) sich in dem Teil des Raums befindet, der an der ersten Auftragfläche (6) mündet, während das zweite Produkt (P') sich in dem Teil des Raums (4) befindet, der an der zweiten Auftragfläche (60) mündet.

7. Applikator nach Anspruch 6, **dadurch gekennzeichnet, daß** das erste und das zweite Produkt (P, P') unterschiedliche Farben aufweisen.

8. Applikator nach Anspruch 1, **dadurch gekennzeichnet, daß** er ein elastisch verformbares Element aufweist, das auf ein steifes oder halbsteifes Organ (11) aufgesetzt ist, dessen erstes Ende (111) einen Boden für den Raum (4) definiert und dessen zweites Ende (110) ein Greifmittel für den Applikator (1) definiert.

9. Applikator nach einem der vorhergehenden Ansprüche, **dadurch gekennzeichnet, daß** das Produkt (P) in dem Raum (oder den Räumen) (4, 40, 41, 42, 43) durch Verankerungsmittel blockiert wird, die sich am Boden und/oder an den Wänden des Raums befinden.

10. Applikator nach einem der Ansprüche 1 bis 9, **dadurch gekennzeichnet, daß** er mindestens einen Schaumstoffblock mit offenen, halboffenen oder geschlossenen Zellen (2, 8, 20) aufweist.

11. Applikator nach Anspruch 10, **dadurch gekennzeichnet, daß** er mindestens einen Schaumstoffblock mit offenen oder halboffenen Zellen aufweist, wobei die Zellen die Verankerungsmittel für das Produkt bilden.

12. Applikator nach Anspruch 10, **dadurch gekennzeichnet, daß** er zwei Schaumstoffblöcke (2, 8) mit offenen oder halboffenen Zellen aufweist, wobei ein erster Block (2) mindestens einen Raum (4) für das Produkt definiert, während ein zweiter Block (8) einen Boden des Raums und eine Fläche (27) definiert, um das mittels der Auftragfläche (6) aufgetragene Produkt zu verwischen, wobei die beiden Blöcke (2, 8) durch einen steifen oder halbsteifen Kern (3) voneinander getrennt sind, wobei der Kern einen Boden für den Raum (4) bildet.

13. Applikator nach Anspruch 10 oder 11, **dadurch gekennzeichnet, daß** er mehrere konzentrische Schaumstoffblöcke (200, 201, 202) aufweist, die um den Raum (4) herum angeordnet sind.

14. Applikator nach Anspruch 10 oder 11, **dadurch gekennzeichnet, daß** er einen Stapel mehrerer Schaumstoffblöcke (200, 201, 202) aufweist, wobei jeder der Schaumstoffblöcke ein ihn von einer Seite zur anderen durchquerendes Loch aufweist, wobei die Schaumstoffblökke so gestapelt sind, daß die Löcher jedes der Schaumstoffblöcke (200, 201, 202) im wesentlichen zueinander in Flucht liegen.

15. Applikator nach einem der vorhergehenden Ansprüche, **dadurch gekennzeichnet, daß** die Auftragfläche (6) in der Ruhestellung des elastisch verformbaren Elements (2) ein kegelstumpfförmiges Profil aufweist, dessen kleinster Durchmesser nahe dem Raum (4) liegt.

16. Applikator nach einem der Ansprüche 1 bis 15, **dadurch gekennzeichnet, daß** die Auftragfläche(n) (6, 60) mit einem Flor (7), einem Textilmaterial oder einem Raster bedeckt ist (sind).

17. Applikator nach einem der vorhergehenden Ansprüche, **dadurch gekennzeichnet, daß** das elastisch verformbare Element (die elastisch verformbaren Elemente) (2, 8) aus einem Schaumstoff aus Polyvinylchlorid (PVC), aus Polyurethan, Polyether, Polyester oder einem Elastomer der Art SBR (synthetischer Butadienkautschuk), NBR, Silikon, Nitril usw. besteht (bestehen).

18. Applikator nach einem der vorhergehenden Ansprüche, **dadurch gekennzeichnet, daß** das Produkt (P, P', P") ein Rouge, eine Wangenschminke, ein Lidschatten, ein Lippenstift, ein Fond de Teint, ein Pflegeprodukt, ein Sonnenschutz- oder ein Haarprodukt usw. ist.

19. Auftrageinheit (10), die aufweist:
a) ein Gehäuse mit einem Boden (12) und einem Deckel (13), der den Boden bedecken kann; und
b) mindestens einen Applikator (1, 1', 1"), der in das Gehäuse eingesetzt ist, **dadurch gekennzeichnet, daß** der Applikator (die Applikatoren) einem der vorhergehenden Ansprüche entspricht (entsprechen).

20. Auftrageinheit nach Anspruch 19, **dadurch gekennzeichnet, daß** der Boden (12) des Gehäuses einen Boden für den Raum (die Räume) (4) des Applikators bildet.

21. Auftrageinheit nach Anspruch 19, **dadurch gekennzeichnet, daß** der Deckel eine Öffnung (18) aufweist, die sich in der geschlossenen Stellung des Deckels (13) auf dem Boden (12) im wesentlichen vor dem Loch (9) des Applikators befindet, um das Gießen des Produkts in den Applikator durch die Öffnung (18) hindurch zu ermöglichen.

22. Auftrageinheit nach Anspruch 21, **dadurch gekennzeichnet, daß** die Öffnung (18) des Deckels nach dem Gießen des Produkts (P) in den Applikator mit einem transparenten Etikett (19) bedeckt wird, damit die Farbe des Produkts (P) im Gehäuse angezeigt werden kann.

## Claims

1. Applicator (1) for spreadable product (P) comprising at least one elastically deformable element (2, 8), at least one face of which constitutes a surface (6, 60) for applying the product, at least one of the elastically deformable elements having at least one hole (9) of axis A, **characterized in that** the hole opens onto the application surface (6) over its entire cross section and delimits at least one open housing (4, 40, 41, 42, 43) in which the product is fixedly arranged, the said product having at least one free surface (5, 50), it being possible for the said elastically deformable element to pass selectively from a position of rest in which the said free surface (5, 50) is level with or beneath the application surface (6, 60) to an application position in which the elastically deformable element (2, 8) is at least partially compressed along the axis A, and in which the said free surface (5, 50) is roughly level with the application surface (6, 60).

2. Applicator according to Claim 1, **characterized in that** the product is poured or compacted into the housing.

3. Applicator according to Claim 1, **characterized in that** the product is mounted in the housing via a cup (12').

4. Applicator according to Claim 1 or 2, **characterized in that** it comprises a surface (3), which is the opposite surface to the application surface (6), the said housing (4) opening only onto the application surface.

5. Applicator according to Claim 1 or 2, **characterized in that** it comprises a second application surface (60) which is on the opposite side to a first application surface (6), the said housing opening both onto the first application surface (6) and onto the second application surface (60).

6. Applicator according to Claim 5, **characterized in that** the housing contains a first and a second product (P, P'), the first product (P) being **in that** part of the housing that opens onto the first application surface (6), the second product (P') being **in that** part of the housing (4) that opens onto the second application surface (60).

7. Applicator according to Claim 6, **characterized in that** the said first and second products (P, P') are different colours.

8. Applicator according to Claim 1, **characterized in that** it comprises an elastically deformable element mounted on a rigid or semi-rigid member (11), a first end (111) of which defines a bottom for the said housing (4), a second end (110) defining a means of holding the said applicator (1).

9. Applicator according to any one of the preceding claims, **characterized in that** the product (P) is immobilized in the housing(s) (4, 40, 41, 42, 43) by anchoring means situated on the bottom and/or on the walls of the housing.

10. Applicator according to any one of Claims 1 to 9, **characterized in that** it comprises at least one block of open-cell, semi-open-cell or closed-cell foam (2, 8, 20).

11. Applicator according to Claim 10, **characterized in that** it comprises at least one block of open-cell or semi-open-cell foam, the said cells constituting the means of anchoring the product.

12. Applicator according to Claim 10, **characterized in that** it comprises two blocks (2, 8) of open-cell or semi-open-cell foam, a first block (2) defining at least one housing (4) for the product, a second block (8) defining a bottom for the said housing and a surface (27) for smudging the product applied using the application surface (6), the two blocks (2, 8) being separated by a rigid or semi-rigid core (3), the said core forming a bottom for the said housing (4).

13. Applicator according to Claim 10 or 11, **characterized in that** it comprises a number of concentric blocks of foam (200, 201, 202) arranged around the housing (4).

14. Applicator according to Claim 10 or 11, **characterized in that** it comprises a stack of a number of blocks of foam (200, 201, 202), each of the blocks of foam having a hole passing right through, the blocks of foam being stacked in such a way that the holes in each of the blocks of foam (200, 201, 202) are practically aligned.

15. Applicator according to any one of the preceding claims, **characterized in that** the application surface (6) has, when the elastically deformable element (2) is in the position of rest, a frustoconical profile, the smallest diameter of which is adjacent to the housing (4).

16. Applicator according to any one of Claims 1 to 15, **characterized in that** the application surface(s) (6, 60) is(are) covered with flocking (7), with a textile material, or with a gauze.

17. Applicator according to any one of the preceding claims, **characterized in that** the elastically deformable element(s) (2, 8) consists (consist) of a foam of polyvinyl chloride (PVC), of polyurethane, of polyether, of polyester, or of an elastomer of the SBR (synthetic butadiene rubber), NBR, silicone, nitrile, etc. type.

18. Applicator according to any one of the preceding claims, **characterized in that** the said product (P, P', P") is a blusher, a rouge, an eyeshadow, a lipstick, a foundation, a care product, a sun care product or a hair product, etc.

19. Application unit (10) comprising:
a) a case having a bottom (12) and a lid (13) capable of covering the said bottom; and
b) at least one applicator (1, 1', 1") mounted in the said case, **characterized in that** the applicator(s) is(are) as per any one of the preceding claims.

20. Application unit according to Claim 19, **characterized in that** the bottom (12) of the case defines a bottom for the housing(s) (4) of the said applicator.

21. Application unit according to Claim 19, **characterized in that** the lid has an orifice (18) which, when the lid (13) is in the closed position over the bottom (12), lies practically facing the hole (9) in the applicator, so that the product can be poured into the applicator through the said orifice (18).

22. Application unit according to Claim 21, **characterized in that** the orifice (18) in the lid is, once the product (P) has been poured into the applicator, covered with a transparent sticker (19) so that the colour of the product (P) in the case can be seen.
